(19) Europäisches Patentamt
European Patent Office
Office européen des brevets

(11) Numéro de publication: **0 134 165**
**B1**

(12) **FASCICULE DE BREVET EUROPÉEN**

(45) Date de publication du fascicule du brevet: **08.06.88**

(21) Numéro de dépôt: **84401391.2**

(22) Date de dépôt: **29.06.84**

(51) Int. Cl.⁴: **C 07 D 401/04**, C 07 D 471/04, A 61 K 31/47, A 61 K 31/435 // C07D215/56 ,(C07D471/04, 221:00, 221:00)

(54) **Dérivés 7-(pyrrol-1-yl) des acides 1-éthyl-1,4-dihydro-4-oxoquinoléine-3-carboxyliques et 1-éthyl-1,4-dihydro-4-oxo-(1,8-naphtyridine)-3-carboxyliques substitués, leur préparation et leur application en tant que médicaments.**

(30) Priorité: **06.07.83 FR 8311250**
**13.02.84 FR 8402145**

(43) Date de publication de la demande:
**13.03.85 Bulletin 85/11**

(45) Mention de la délivrance du brevet:
**08.06.88 Bulletin 88/23**

(84) Etats contractants désignés:
**AT BE CH DE FR GB IT LI LU NL SE**

(56) Documents cité:
**EP-A-0 009 425**
**FR-A-2 210 413**

**CHEMICAL ABSTRACTS, vol. 87, 1977, page 551, no. 68328q Columbus, Ohio, US**

(73) Titulaire: **PROVESAN S.A., 19, rue de la Croix d'Or, CH- 1211 Genève (CH)**

(72) Inventeur: **Esteve Soler, José, Via Augusta 244, Barcelone (ES)**

(74) Mandataire: **Ahner, Francis, CABINET REGIMBEAU 26, avenue Kléber, F-75116 Paris (FR)**

EP 0 134 165 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

## Description

La présente invention se rapporte à de nouveaux dérivés des acides 1-éthyl-1,4-dihydro-4-oxoquinoléine-3-carboxyliques et 1-éthyl-1,4-dihydro-4-oxo-(1,8-naphtyri-din)-3-carboxyliques, tous deux substitués en position 7 par le groupe pyrrol-1-yl, ainsi qu'à leur préparation et à leur application en tant que médicaments.

L'état de la technique le plus proche peut être illustré par FR-A-2 210 413 et EP-A-0 009 425 décrivant des dérivés de pipérazine qui se distinguent très clairement des composés de la présente invention par leur substitution en position 7.

Les nouveaux dérivés, objet de la présente invention, répondent à la formule générale I :

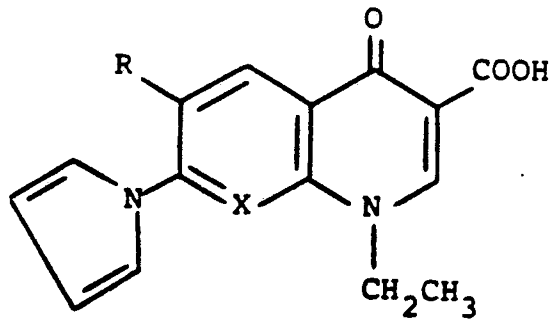

(I)

dans laquelle :

X représente le groupe CH ou un atome d'azote, et

R représente un atome d'hydrogène ou un atome de fluor.

La présente Invention se rapporte également aux sels alcalins ou alcalino-terreux physiologiquement acceptables des composés de formule générale I.

Les dérivés de formule générale I et leurs sels présentent d'intéressantes propriétés pharmacologiques antimicrobiennes en particulier, antibactériennes et fongistatiques.

Les nouveaux composés présentent une puissante activité antibactérienne à la fois à l'égard des bactéries Gram-positives et des bactéries Gram-négatives.

Les nouveaux dérivés de formule générale I peuvent être préparés conformément à l'invention, selon le schéma réactionnel suivant :

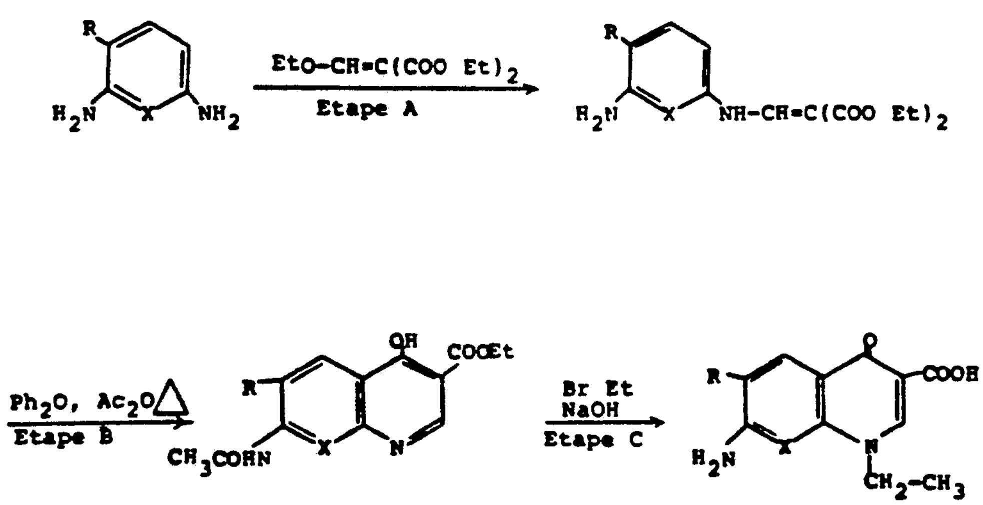


(II)

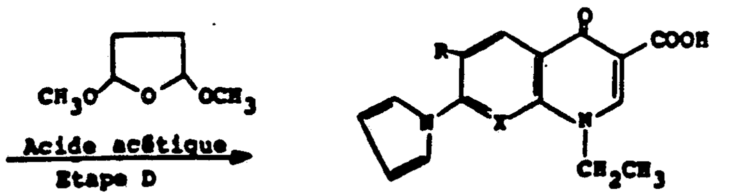


(I)

où X et R ont les significations données précédemment.

A l'étape A, la diamine correspondante est condensée directement avec l'éthoxyméthylène malonate de diéthyle pour donner le monoaminométhylène malonate d'éthyle par élimination d'alcool. Au cours de l'étape B, le composé est cyclisé par chauffage soit en absence de solvant, soit moyennant l'emploi d'un solvant approprié servant d'échangeur thermique comme par exemple le benzène, le toluène, le xylène, la tétraline, le nitrobenzène, le dichlorobenzène, le diphényléther ou le biphényle, ou encore un mélange de ces solvants.

La température de réaction est comprise entre 150° C et 250° C, de préférence entre 180-230° C. En employant certains catalyseurs on peut obtenir la cyclisation à des températures beaucoup plus basses. Parmi les catalyseurs appropriés on peut citer à titre d'exemple l'ester polyphosphorique, l'acide polyphosphorique et l'anhydride phosphorique. Avec ces catalyseurs on utilise des températures comprises généralement entre 60-170° C, mieux encore entre 75° C et 150° C.

Puis au cours de l'étape C, on prépare les composés N-alkylés. On peut effectuer l'alkylation en utilisant l'un des agents d'alkylation classiques, lesquels comprennent les halogénures d'alkyle, les sulfates dialkyliques et les sulfonates d'alkyle.

En général, la réaction s'effectue en présence d'un alcali et dans un solvant inerte vis-à-vis de la réaction. Les solvants peuvent notamment être constitués par l'eau, le méthanol, l'éthanol, l'acétone, le dioxane, le benzène, le diméthylformamide, le diméthylsulfoxyde, ainsi que des mélanges de ces solvants.

Les alcalis préférés qui peuvent être utilisés sont les hydroxydes alcalins, tels que l'hydroxyde de sodium et l'hydroxyde de potassium, ou bien des carbonates alcalins, tels que le carbonate de sodium ou le carbonate de potassium.

Il convient de noter qu'au cours de l'étape C, le procédé d'alkylation s'accompagne d'une hydrolyse de l'ester carboxylique, puisque le milieu est franchement alcalin, et l'on obtient donc les acides carboxyliques correspondants.

A l'étape ultime D, on procède au greffage du noyau pyrrole selon la méthode de Clauson-Kaas, Acta Chem. Scand. 6, 667 et 867 (1952), par réaction de l'amine et du diméthoxytétrahydrofurane, par reflux pendant 1/2 heure en milieu acide acétique.

Dans le cas particulier de la préparation de l'acide 1-éthyl-1,4-dihydro-4-oxo-6-fluoro-7-(pyrrol-l-yl)-1,8-naphtyridine-3-carboxylique, il convient de remarquer que l'intermédiaire de synthèse, nécessaire à la mise en oeuvre de l'étape D de greffage du noyau pyrrole, est nouveau.

Dans ce cas particulier, cette étape réactionnelle D se schématise comme suit :

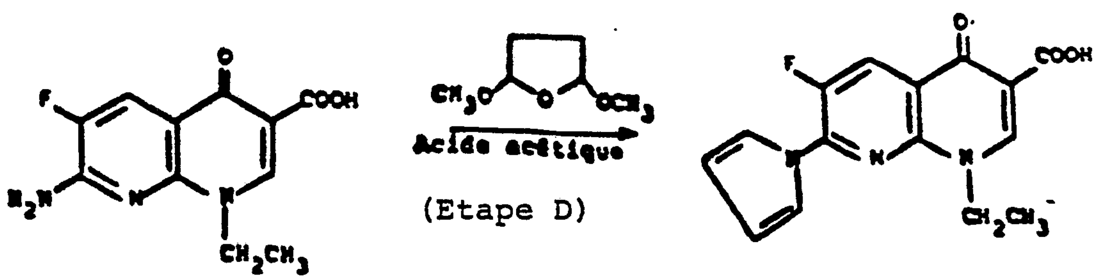


(IIa) (Ia)

Cet intermédiaire de synthèse peut par exemple être préparé à partir de l'acide 1-éthyl-1,4-dihydro-4-oxo-6-fluoro-7-chloro-1,8-naphtyridine-3-carboxylique (par exemple décrit dans EP-A-0 027 752) conformément au schéma réactionnel suivant :

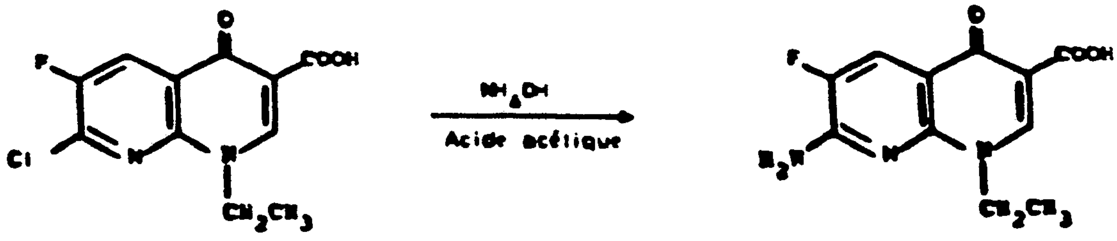


(IIa)

Dans les exemples suivants on indiquera la préparation de nouveaux dérivés selon l'invention ainsi que des matières de départ correspondantes et des produits intermédiaires. On décrira en outre quelques formes d'emploi typiques pour les différents domaines d'application.

Les exemples ci-après sont donnés à simple titre d'illustration.

**EXEMPLE 1**

Préparation du 3-amino-anilin-méthylènemalonate de diéthyle (étape A)

On dissout 10,8 grammes de m-phénylèndiamine dans 80 ml d'alcool éthylique, on ajoute 21,6 grammes d'éthoxyméthylènmalonate de diéthyle et on chauffe à reflux pendant 40 minutes. On filtre à chaud, on ajoute 50 ml d'eau et on laisse à température ambiante avec agitation pendant 24 à 36 heures. On filtre le précipité formé, on le lave avec un mélange éthanol/eau (1 : 1) et on sèche à 60°C. On recristallise avec un mélange benzène/hexane (2 : 1) et on obtient 10,5 grammes d'un solide de point de fusion 71-74°C.

Préparation du 7-acétamido-4-hydroxy-3-quinoléincarboxylate d'éthyle (étape B)

On dissout 10,5 grammes de 3-amino-anilinméthylèn-malonate de diéthyle dans 80 ml d'oxyde de diphényle, on ajoute 8 ml d'anhydride acétique, on chauffe progressivement jusqu'à 250°C et on maintient à reflux pendant 10 minutes. On laisse refroidir, on ajoute 20 ml d'éthanol, on filtre et on lave avec de l'éthanol. On recristallise avec du diméthylformamide et on obtient 4,6 grammes d'un solide de point de fusion 295-300°C.

Préparation de l'acide 7-amino-1-éthyl-1,4-dihydro-4-oxo-3-quinoléincarboxylique (étape C)

On dissout 4,6 grammes de 7-acétamido-4-hydroxy-3-quinoléincarboxylate d'éthyle dans 15 ml de NaOH à 10 %, 60 ml de $H_2O$ et 100 ml d'éthanol et on ajoute 5 ml de bromure d'éthyle. On laisse à reflux pendant 4 heures, puis on évapore l'excès de bromure d'éthyle et d'éthanol, puis on ajoute 10 ml de NaOH à 10 %. On chauffe à reflux pendant 2 heures, on laisse refroidir, on acidifie avec HCl, on filtre et on traite avec de l'éthanol à 70°C. On filtre et on recristallise avec un mélange diméthylformamide-eau (1 : 1). On obtient 1,0 gramme d'un solide de point de fusion 304-307°C.

Préparation de l'acide 7-(pyrrol-1-yl)-1-éthyl-1,4-dihydro-4-oxo-3-quinoléincarboxylique (étape D)

On met en suspension 0,3 gramme d'acide 7-amino-1-éthyl-1,4-dihydro-4-oxo-3-quinoléincarboxylique dans 10 ml d'acide acétique, on ajoute 0,17 gramme de diméthoxytétrahydrofurane et on chauffe jusqu'à dissolution. On filtre et on ajoute au filtrat de l'eau jusqu' à obtention d'un trouble. On laisse refroidir, on filtre le précipité obtenu et on lave avec de l'éthanol. On obtient 0,12 gramme d'un solide de point de fusion 235-238°C.

Données spectroscopiques:

1H RMN, δ, [DMSO ($d_6$)] ; 1,46 (t,3H) ; 4,57 (q, 2H) ; 6,23 (m,2H) ; 7,43 (m,2H) ; 7,59 [d(J=8 Hz), 1H] ;7,68 (s,1H) ; 8,18 [d(J = 8 Hz) ,1H] ; 8,76 (s,1H) ; 14,80 (s,1H). IR (KBr) : 1620, 1720 $cm^{-1}$.

**Exemple 2**

Préparation de la 4-fluoro-m-phénylèndiamine

A une solution de 9 grammes de $Cl_2S_n.2H_2O$ dans 12 ml de HCl concentré, on ajoute sous agitation en une fois 1,6 grammes de 4-fluoro-3-nitroaniline qui se dissout en produisant une vive réaction qui atteint 95-100°C. On laisse refroidir à température ambiante et on verse le contenu sur une solution de 70 ml de NaOH à 50 % dans de la glace, de façon que la température reste inférieure à 20°C. On extrait la solution fortement alcaline qui en résulte trois fois avec 50 ml d'éther éthylique. On réunit les extraits d'éther éthylique, on les lave avec 30 ml d'eau distillée et on les sèche avec du sulfate de sodium anhydre. On évapore à sec la solution d'éther éthylique et on obtient 1,2 gramme d'une huile de couleur sombre.

Préparation du 4-fluoro-3-amino-anilinméthylènmalonate de diéthyle (étape A)

On chauffe à reflux pendant 30 minutes une solution de 2,16 qrammes d'éthoxyméthylènmalonate de diéthyle et 1,26 gramme de 4-fluoro-m-phénylèndiamine dans 40 ml d'éthanol et on ajoute à chaud 15 ml d'eau.

On laisse refroidir et on filtre le précipité formé que l'on lave avec un mélange éthanol/$H_2O$ (1 : 1) On sèche à 60° C, on recristallise avec un mélange benzène/hexane (2 : 1) et on obtient 1,6 gramme de cristaux de point de fusion 100-102° C.

Préparation du 7-acétamido-4-hydroxy-6-fluoro-3-quinoléin-carboxylate d'éthyle (etape B)

On dissout 1,6 gramme de 4-fluoro-3-amino-anilinméthylènmalonate de diéthyle dans un mélange de 8 ml d'oxyde de diphényle et 1 ml d'anhydride acétique et on chauffe progressivement jusqu' à 250° C, température à laquelle apparaît un précipité. On laisse 10 minutes à reflux et on laisse refroidir. On ajoute 5 ml d'éthanol, on filtre et on lave à l'éthanol. On recristallise avec du diméthylformamide et on obtient 1 gramme d'un solide de point de fusion 320° C.

Préparation de l'acide 6-fluoro-7-amino-1-éthyl-1,4-dihydro-4-oxo-3-quinoléincarboxylique (étape C)

A une solution de 1 gramme de 7-acétamido-4-hydroxy-6-fluoro-3-quinoléincarboxylate d'éthyle dans 25 ml d'eau, 60 ml d'éthanol et 2,5 ml d'hydroxyde de sodium à 10 %, on ajoute 1,5 ml de bromure d'éthyle et on maintient à reflux pendant 4 heures. Ensuite on concentre à la moitié du volume, on ajoute 5 ml d'hydroxyde de sodium à 10 % et on maintient à reflux pendant 1 heure. On laisse refroidir, on acidifie avec de l'acide chlorhydrique et on filtre le précipité formé. On lave le précipité à l'eau, on sèche et on recristallise avec un mélange diméthylformamide/eau (10 : 1)

On obtient 0,65 gramme d'un solide qui fond à 298-300° C avec décomposition.

Préparation de l'acide 6-fluoro-7- (pyrrol-1 -yl)-1-éthyl-1,4-dihydro-4-oxo-3-quinoléincarboxylique (étape D)

On suspend 2,5 grammes d'acide 6-fluoro-7-amino-1-éthyl-1,4-dihydro-4-oxo-3-quinoléincarboxylique dans 15 ml d'acide acétique et on ajoute 1,32 gramme de diméthoxytétrahydrofurane ; on chauffe progressivement jusqu' à dissolution, puis on laisse refroidir. On filtre le précipité formé et on lave à l'éthanol. On recristallise avec de l'acétonitrile et on obtient 1,4 gramme d'aiguilles de point de fusion 251-252° C.

Données spectroscopiques:

$^1H$ RMN, δ, [DMSO ($d_6$)] ; 1,48 (t,3H) ; 4,62 (q,2H) ; 6,38 (t,2H) ; 7,34 (q,2H) ; 7,99 [d(J = 6,1 Hz) , 1H] ; 8,10 [d(J = 11,4 Hz), 1H] ; 8,92 (s,1H) ; 14,65 (s,1H) IR (KBr) : 1620, 1720 $cm^{-1}$.

**Exemple 3**

Préparation de l'acide 1-éthyl-1,4-dihydro-4-oxo-7-(pyrrol-1-yl)-1,8-naphtyridin-3-carboxylique (étape D)

On met à reflux pendant 30 minutes 4,6 grammes d'acide 1-éthyl-1,4-dihydro-4-oxo-7-amino-1,8-naphtyridin-3-carboxylique (brevet US 3 149 104) et 2,7 grammes de 2,5-diméthoxytétrahydrofurane dans 70 ml d'acide acétique glacial. On laisse refroidir, puis on laisse pendant 8 heures à 5° C et on obtient un précipité qui, filtré et recristallisé dans l'acétonitrile, donne 4,3 grammes d'aiguilles de point de fusion 230-232° C.

Données spectroscopiques:

$^1H$ RMN, δ, [DMSO ($d_6$)] ; 1,47 (t, 3H) ; 4,57 (q,2H) ; 6,30 (m,2H) ; 7,70 (m,2H) ; 7,80 [d(J = 8,4 Hz),1H] ; 8,53 [d (J = 8,4 Hz), 1H] ; 8,95 (s,1H) ; 14,62 (s,1H) IR (KBr) : 1625, 1720 $cm^{-1}$.

**Exemple 4**

Préparation de l'acide 1-éthyl-1,4-dihydro-4-oxo-6-fluoro-7- (pyrrol-1-yl)-1,8-naphtyridine-3-carboxylique (étape D)

On met en suspension 1,4 gramme d'acide 1-éthyl-1,4-dihydro-4oxo-6-fluoro-7-amino-1,8-naphtyridine-3-carboxylique de point de fusion 299-303° C (décomp.) et ayant les données spectroscopiques suivantes :

$^1H$ RMN, δ [$CF_3COOH$] : 1,70 (t,3H) ; 4,83 (q,2H) ; 8,10 [d(j=9,4Hz), 1H] ; 9,11 (s,1H). IR (KBr) : 1650, 1720, 3320, 3425 $cm^{-1}$

dans 20 ml d'un mélange d'acide acétique et de diméthylformamide (1 : 1). On ajoute 0,8 gramme de 2,5-diméthoxytétrahydrofurane et on chauffe au reflux pendant 10 minutes. On laisse refroidir puis on laisse reposer pendant 8 heures à 5° C et on obtient un précipité qui, filtré et recristallisé dans l'acétone, donne 0,95 gramme de cristaux aciformes de point de fusion 257-259° C.

Données spectroscopiques:

$^1H$ RMN, δ, [$CF_3COOH$] : 1,67 (t, 3H) ; 4,88 (q, 2H) ; 6,36 (m,2H) ; 7,68 (m,2H) ; 8,40 [d(j=11Hz),1H] ; 9,23 (s,1H) IR (KBr) : 1625, 1725 $cm^{-1}$.

Le composé de départ peut être préparé comme suit : 1 gramme d'acide 1-éthyl-1,4-dihydro-4-oxo-6-fluoro-7-chloro-1,8-naphtyridine-3-carboxylique (par exemple décrit dans EP-A-0 027 752) est mélangé avec 25 ml d'ammoniaque concentrée contenant 20 % d'éthanol. On maintient le mélange dans un tube scellé à 120-125° C pedant 4 heures. On refroidit et on ajoute de l'acide acétique jusqu' à acidité légère et le précipité formé est filtré et lavé avec de l'eau. On sèche et on obtient 0,8 qramme d'acide 1-éthyl-1,4-dihydro-4-oxo-6-fluoro-7-amino-1,8-naphtyridine-3-carboxylique de point de fusion 299-303° C.

Activité pharmacologique antimicrobienne (G.L. Daquet et Y. A. Chabbect, Techniques en bactèriologie, vol 3, Flammarion Medecine-Sciences, Paris, 1972 et W.B. Hugo et A.D. Rusell, Pharmaceutical Microbiology,

Blackwell Scientific Publications, London, (1977).

- Milieu de culture et solvant :

Agar d'antibiotiques n° 1 (Oxoid CM 327) Bouillon de triptone-soja (Oxoid CM 129) Solution physiologique Ringer 1/4 (Oxoid BR 52) Agar Dextrose (BBL-11165) NaOH 0,1 N

- Microorganismes :

"Bacilus subtilis" ATCC 6633
"Citrobacter freundii" ATCC 11606
"Enterobacter aerogenes" ATCC 15038
"Enterobacter cloacae" CHSP 20
"Escherichia coli" ATCC 10536
"Escherichia coli" R-1513
"Klebsiella pneumoniae" ATCC 10031
"Micrococcus flavus" ATCC 10240
"Proteus mirabilis" ATCC 4675
"Proteus morganii" CHSP 16
"Pseudomonas aeruginosa" ATCC 25115
"Pseudomonas aeruginosa" ADSA 47
"Salmonella tiphymurium" AMES 98
"Salmonella tiphymurium" AMES 100
"Sarcina lutea" ATCC 9341
"Serratia marcescens" ATCC 13880
"Shigella flexnerii" ATCC 12022
"Staphylococcus aureus" ATCC 5488/23
"Staphylococcus aureus" ATCC 25178
"Streptococcus faecalis" ATCC 10541

- Préparation des inoculations

Chacun des microorganismes est ensemencé par strie dans des tubes d'Agar d'antibiotiques n°1, et mis en incubation pendant 20 heures à 37°C. Ensuite on prend une anse de culture, on ensemence dans un bouillon de Triptone-soja et on incube pendant 20 heures à 37°C. On dilue à 1/4 la culture obtenue avec une solution physiologique Ringer, de façon à obtenir une suspension normalisée de $10^7$-$10^9$ ufc/ml pour chaque organisme.

- Préparation du milieu contenant les dérivés de formule générale 1

A partir d'une solution de 1000 µg/ml dans NaOH 0,1 N, chaque produit est dilué dans l'Agar Dextrose (préalablement fondu et maintenu à 50°C) par dilutions successives de façon à obtenir les concentrations suivantes : 64 - 32 - 16 - 8 - 4 - 2 - 1 - 0,5 - 0,25-0,125 µg de dérivé/ml du milieu.

Postérieurement, chaque concentration de chaque produit est répartie dans des boîtes de Petri de 10 cm de diamètre, à raison de 10 ml de milieu par boîte et autant de boîtes que de microorganismes à tester.

Une fois le milieu refroidit, les boîtes sont ensemencées avec les inoculations à raison de 0,4 ml d'inoculation par boîte. On les étend avec une anse de Driglasky et on recueille le surnageant. Les boîtes ensemencées sont incubées à 37°C pendant 20 heures.

Résultats

Les résultats obtenus sont décrits dans le tableau 1. Les produits des exemples 1,2 et 4 ont une activité "in vitro" supérieure à celle de l'acide pipémidique, tant à l'égard des Enterobacteriaceas, (pseudomonas aeruginosa) que des coques Gram-positifs. Le dérivé de l'exemple 3 présente une activité du même ordre que l'acide pipémidique face aux microorganismes Gram-négatifs et une activité supérieure face aux coques Gram-positifs.

**TABLEAU 1**
CMI "in vitro" comparée à l'acide pipémidique
Les concentrations sont données en µ/ml.

| MICROORGANISMES | Composé de l'EXEMPLE 1 | Composé de l'EXEMPLE 2 | Composé de l'EXEMPLE 3 | Composé de L'EXEMPLE 4 | ACIDE PIPEMIDIQUE |
|---|---|---|---|---|---|
| Bacillus subtilis ATCC 6633 | <0,125 | <0,125 | 0,25 | 0,03 | 8 |
| Citrobacter freundii ATCC 11606 | 16 | 8 | 32 | 4,00 | 4 |
| Enterobacter aerogenes ATCC 15038 | >64 | 8 | >64 | 4,00 | 32 |
| Enterobacter cloacae CHSP 20 | 16 | 1 | 8 | 2,00 | 8 |
| Escherichia coli ATCC 10536 | 4 | 1 | 8 | 0,12 | 2 |
| Escherichia coli R-1513 | 16 | 4 | 16 | 4,00 | 16 |
| Klebsiella pneumoniae ATCC 10031 | 1 | 0,5 | 4 | 1,00 | 2 |
| Micrococcus flavus ATCC 10240 | 16 | 8 | 4 | 1,00 | >64 |
| Proteus mirabilis ATCC 4675 | 16 | 4 | >64 | 8,00 | 16 |
| Proteus morganii CHSP 16 | 8 | 2 | 8 | 4,00 | 8 |
| Pseudomonas aeruginosa ATCC 25115 | >64 | 16 | >64 | 32,00 | 32 |
| Pseudomonas aerguinosa ADSA 47 | >64 | 64 | >64 | >64,00 | 32 |
| Salmonella tiphymurium AMES 98 | 0,5 | <0,125 | 0,5 | 0,12 | 4 |
| Salmonella tiphymurium AMES 100 | 4 | 0,5 | 8 | 0,50 | 8 |
| Sarcina lutea ATCC 9341 | 16 | 16 | 8 | 4,00 | >64 |
| Serratia marcescens ATCC 13880 | 8 | 2 | 16 | 2,00 | 16 |
| Shigella flexnerii ATCC 12022 | 8 | 2 | 16 | 2,00 | 4 |
| Staphylococcus aureus ATCC 5488/23 | 1 | 0,25 | 8 | 0,50 | 64 |
| Staphylococcus aureus ATCC 25178 | 1 | 0,25 | 4 | 0,50 | 64 |
| Streptococcus faecalis ATCC 10541 | 16 | 1 | 32 | 8,00 | >64 |

Toxicité aiguë chez la souris

Pour déterminer cette toxicité, on a utilisé comme animaux d'expérimentation des souris albinos de souche C.F.L.P. des deux sexes et d'un poids compris entre 19 et 25 grammes. Après une période de jeûne de 18 heures avec de l'eau "ad libitum", on administre les dérivés objets de la présente invention en suspension dans la gomme arabique à 5 % par voie intrapéritonéale. Le volume de la suspension administrée a été dans tous les cas de 0,4 ml/20 grammes (20 ml/kg), en changeant la concentration de la suspension selon la dose administrée

Une heure après l'administration des dérivés, on fournit aux animaux une nourriture standard rat-souris Panlab. La période d'observation de la mortalité a été de 7 jours. Avec aucun des produits on a observé des différences de mortalité entre les sexes.

Les résultats obtenus sont décrits dans la tableau II.

**TABLEAU II**

| Dérivés | voie d'administration | | DL-50 mg/kg |
|---|---|---|---|
| Exemple 1 | i.p. | > | 800 |
| Exemple 2 | i.p. | > | 1600 |
| Exemple 3 | i.p. | | 900 |
| Exemple 4 | i.p. | > | 1000 |
| Acide nalidixique | i.p. | | 600 |
| Acide pipémidique | i.p. | > | 1600 |

Compte tenu de leurs bonnes propriétés pharmacologiques, les dérivés de formule générale I sont donc susceptibles d'être utilisés en médecine humaine et/ou vétérinaire, pour le traitement des infections aiguës, chroniques et récidivantes systémiques ou localisées, causées par des microorganismes Gram-positifs et Gram-négatifs sensibles aux produits objet de la présente invention, dans le tractus gastro-intestinal ou génitourinaire, l'appareil respiratoire, la peau et les tissus mous, ainsi que les infections neurologiques et odontostomatologiques.

En thérapeutique humaine, la dose proposée des dérivés de la présente invention est environ comprise entre 400 et 1200 mg/jour pour un adulte, par exemple administrée sous la forme de comprimés ou de gélules. Cette posologie peut toutefois varier en fonction de la gravité de l'affection.

On indiquera ci-après, à titre d'exemples, deux formes galéniques particulières des dérivés, objet de la présente invention.

Exemple de formule par comprimé

| | | |
|---|---|---|
| Acide 6-fluoro-7- (pyrrol-1-yl)-1-éthyl-1,4-dihydro-4-oxo-3-quinoléincarboxylique | 0,400 | g |
| Carboxyméthylamidon | 0,018 | g |
| Polyvinylpyrrolidone K29-32 | 0,030 | g |
| Cellulose microcristalline | 0,146 | g |
| Dioxyde silice colloïdale | 0,003 | g |
| Stéarate de magnésium | 0,003 | g |
| | 0,600 | g |

Exemple de formule par gélule

| | | |
|---|---|---|
| Acide 6-fluoro-7-(pyrrol-1-yl)-1-éthyl-1,4-dihydro-4-oxo-3-quinoléincarboxylique | 0,400 | g |
| Cellulose microcristalline | 0,0356 | g |
| Dioxyde silice colloïdale | 0,0022 | g |
| Stéarate de magnésium | 0,0022 | g |
| | 0,440 | g |

**Revendications** pour les Etats contractants

BE CH DE FR GB IT LI LU NL SE

1. Les dérivés des acides 7-(pyrrol-1-yl)-1-éthyl-1,4-dihydro-4-oxo-quinoléin-3-carboxylique et 7- (pyrrol-1-yl)-1-éthyl-1,4-dihydro-4-oxo-(1,8-naphtyri-din)-3-carboxylique selon la formule générale I :

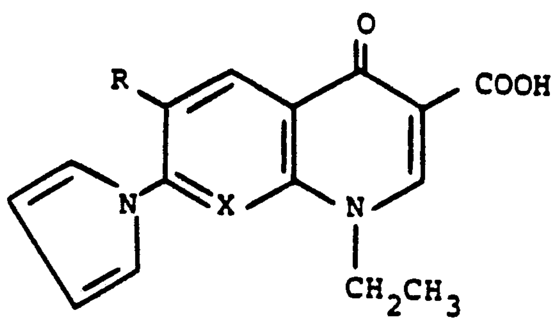

(I)

dans laquelle

X représente le groupe CH ou un atome d'azote, et
R représente un atome d'hydrogène ou un atome de fluor,
ainsi que leurs sels alcalins ou alcalino-terreux physiologiquement acceptables.

2. L'acide 7-(pyrrol-1-yl)-1-éthyl-1,4-dihydro-4-oxo-quinoléin-3-carboxylique répondant a la formule générale I selon la revendication 1.

3. L'acide 7-(pyrrol-1-yl)-6-fluoro-1-éthyl-1,4-dihydro-4-oxo-quinoléin-3-carboxylique répondant à la formule générale I selon la revendication 1.

4. L'acide 7-(pyrrol-1-yl)-1-éthyl-1,4-dihydro-4-oxo-(1,8-naphtyridin)-3-carboxylique répondant à la formule générale I selon la revendication 1.

5. L'acide 7-(pyrrol-1-yl)-6-fluoro-1-éthyl-1,4-dihydro-4-oxo-(1,8-naphtyridine)-3-carboxylique répondant à la formule générale I selon la revendication 1.

6. Procédé de préparation des dérivés de formule générale I tels que définis dans l'une des revendications 1 à 5, caractérisé par le fait que l'on fait réagir un composé de formule générale II

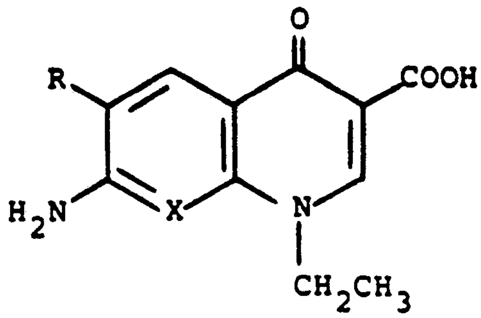

(II)

dans laquelle les symboles X et R ont les significations données à la revendication 1, avec le 2,5-diméthoxytétrahydrofurane en milieu acide acétique.

7. A titre de médicaments nouveaux, spécialement comme antibactériens et fongicides, les dérivés de formule générale I selon l'une des revendications 1 à 5.

8. Compositions pharmaceutiques, caractérisées par le fait qu'elles contiennent, outre un support pharmaceutiquement acceptable, au moins un dérivé de formule générale I selon l'une des revendications 1 à 5.

**Revendications** pour l'Etat contractant AT

1. Procédé de préparation des dérivés des acides 7-(pyrrol-1-yl)-1-éthyl-1,4-dihydro-4-oxo-quinoléin-3-carboxylique et 7-(pyrrol-1-yl)-1-éthyl-1,4-dihydro-4-oxo-(1,8-naphtyridin)-3-carboxylique selon la formule générale 1:

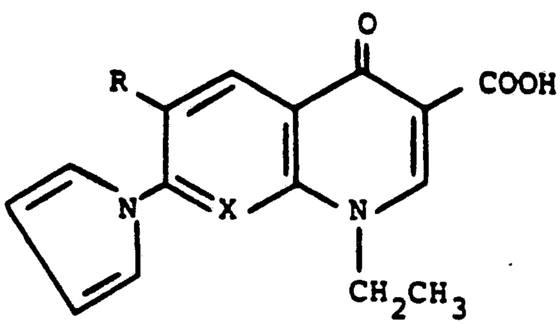

(I)

dans laquelle

X représente le groupe CH ou un atome d'azote, et

R représente un atome d'hydrogène ou un atome de fluor,

ainsi que leurs sels alcalins ou alcalino-terreux physiologiquement acceptables, caractérisé par le fait que l'on fait réagir un composé de formule générale II :

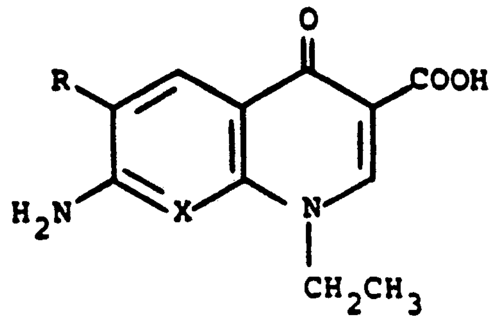

(II)

dans laquelle les symboles X et R ont les significations données ci-dessus, avec le 2,5-diméthoxytétrahydrofurane en milieu acide acétique.

2. Procédé selon la revendication 1 caractérisé en ce que l'on prépare l'acide 7-(pyrrol-1-yl)-1-éthyl-1,4-dihydro- 4-oxo-quinoléin-3-carboxylique répondant à la formule générale I.

3. Procédé selon la revendication 1 caractérisé en ce que l'on prépare l'acide 7-(pyrrol-1-yl)-6-fluoro-1-éthyl-1,4-dihydro-4-oxo-quinoléin-3-carboxylique répondant à la formule générale I.

4. Procédé selon la revendication 1 caractérisé en ce que l'on prépare l'acide 7-(pyrrol-1-yl)-1-éthyl-1,4-dihydro-4-oxo-(1,8-naphtyridin)-3-carboxylique répondant à la formule générale I.

5. Procédé selon la revendication 1 caractérisé en ce que l'on prépare l'acide 7-(pyrrol-1-yl)-6-fluoro-1-éthyl-1,4-dihydro-4-oxo-(1,8-naphtyridin)-3-carboxylique répondant à la formule générale I.

**Patentansprüche** für die Vertragsstauten: BE CH DE FR GB IT LI LU NL SE

1. Derivate der 7-(Pyrrol-1-yl)-1-ethyl-1,4-dihydro-4-oxo-chinolin-3-carbonsäure und der 7-(Pyrrol-1-yl)-1-ethyl-1,4-dihydro-4-oxo-(1,8-naphtyridin)-3-carbonsäure der allgemeinen Formel I

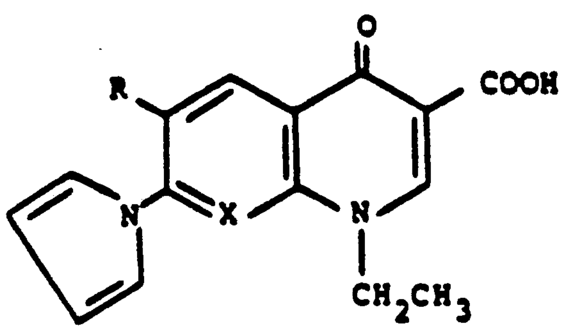

(I)

worin bedeuten:
X die Gruppe CH oder ein Stickstoffatom und
R ein Wasserstoffatom oder ein Fluoratom,
sowie ihre physiologisch akzeptablen Alkali- oder Erdalkalisalze.

2. 7-(Pyrrol-1-yl)-1-ethyl-1,4-dihydro-4-oxo-chinolin-3-carbonsäure der allgemeinen Formel I gemäß Anspruch 1.

3. 7-(Pyrrol-1-yl)-6-fluoro-1-ethyl-1,4-dihydro-4-oxo-chinolin-3-carbonsäure der allgemeinen Formel I gemäß Anspruch 1.

4. 7-(Pyrrol-1-yl)-1-ethyl-1,4-dihydro-4-oxo-(1,8-naphty-ridin)-3-carbonsäure der allgemeinen Formel I gemäß Anspruch 1.

5. 7-(Pyrrol-1-yl)-6-fluoro-1-ethyl-1,4-dihydro-4-oxo-(1,8-naphtyridin)-3-carbonsäure der allgemeinen Formel I gemäß Anspruch 1.

6. Verfahren zur Herstellung der Derivate der allgemeinen Formel I, wie sie in einem der Ansprüche 1 bis 5 definiert sind, dadurch gekennzeichnet, daß man eine Verbindung der allgemeinen Formel II

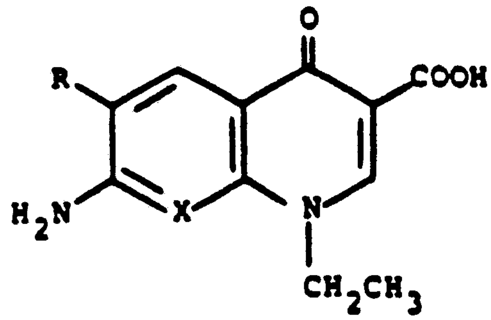

(II)

in der die Symbole X und Y die im Anspruch 1 angegebenen Bedeutungen haben,
mit 2,5-Dimethoxytetrahydrofuran in essigsaurem Milieu reagieren läßt.

7. Als neue Arzneimittel, insbesondere als antibakterielle und fungizide Mittel, die Derivate der allgemeinen Formel I gemäß einem der Ansprüche 1 bis 5.

8. Pharmazeutische Zusammensetzungen, dadurch gekennzeichnet, daß sie außer einem pharmazeutisch akzeptablen Träger mindestens ein Derivat der allgemeinen Formel I gemäß einem der Ansprüche 1 bis 5 enthalten.

**Patentansprüche** Für den Vertragsstaut: AT

1. Verfahren zur Herstellung von Derivaten der 7-(Pyrrol-1-yl)-1-ethyl-1,4-dihydro-4-oxo-chinolin-3-carbonsäure und der 7-(Pyrrol-1-yl)-1-ethyl-1,4-dihydro-4-oxo-(1,8-naphty-ridin)-3-carbonsäure der allgemeinen Formel I

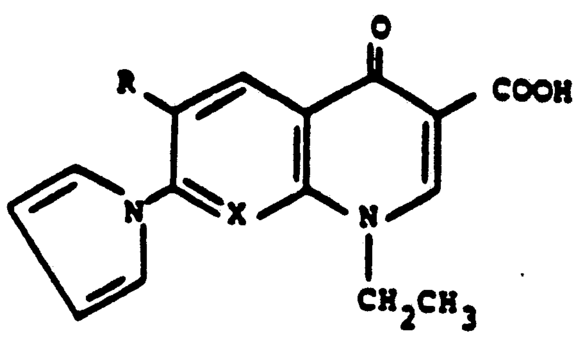

(I)

worin bedeuten:
X die Gruppe CH oder ein Stickstoffatom und
R ein Wasserstoffatom oder ein Fluoratom, sowie ihre physiologisch akzeptablen Alkali- oder Erdalkalisalze, dadurch gekennzeichnet, daß man eine Verbindung der allgemeinen Formel II

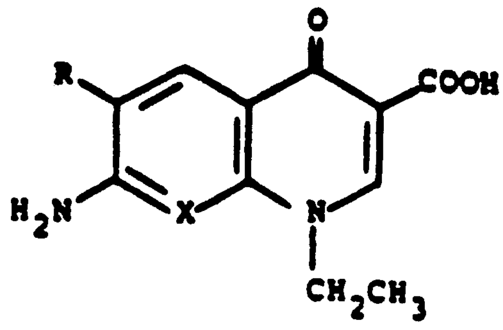

(II)

in der die Symbole X und R die oben angegebenen Bedeutungen haben, mit 2,5-Dimethoxytetrahydrofuran in essigsaurem Milieu reagieren läßt.

2. Verfahren nach Anspruch 1, dadurch gekennzeichnet, daß man die 7-(Pyrrol-1-yl)-1-ethyl-1,4-dihydro-4-oxo-chinolin-3-carbonsäure der allgemeinen Formel I herstellt.

3. Verfahren nach Anspruch 1, dadurch gekennzeichnet, daß man die 7-(Pyrrol-1-yl)-6-fluoro-1-ethyl-1,4-dihydro-4-oxo-chinolin-3-carbonsäure der allgemeinen Formel I herstellt.

4. Verfahren nach Anspruch 1, dadurch gekennzeichnet, daß man die 7-(Pyrrol-1-yl)-1-ethyl-1,4-dihydro-4-oxo-(1,8-naphtyridin)-3-carbonsäure der allgemeinen Formel I herstellt.

5. Verfahren nach Anspruch 1, dadurch gekennzeichnet, daß man die 7-(Pyrrol-1-yl)-6-fluoro-1-ethyl-1,4-dihydro-4-oxo-(1,8-naphtyridin)-3-carbonsäure der allgemeinen Formel I herstellt.

**Claims** For the contracting States: BE CH DE FR GB IT LI LU NL SE

1. The derivatives of 7-(1-pyrrolyl)-1-ethyl-1,4-dihydro-4-oxoquinoline-3-carboxylic acid and 7-(1-pyrro-lyl)-1-ethyl-1,4-dihydro-4-oxo-1,8-naphthyridine-3-carboxylic acid according to the general formula I:

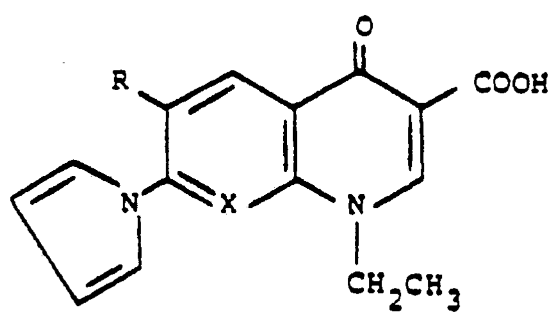

(I)

in which

X represents the CH Group or a nitrogen atom, and

R represents a hydrogen atom or a fluorine atom, as well as their physiologically acceptable alkali metal salts or alkaline earth metal salts.

2. 7-(1-Pyrrolyl)-1-ethyl-1,4-dihydro-4-oxoquino-line-3-carboxylic acid corresponding to the general formula I as claimed in claim 1.

3. 7-(1-Pyrrolyl)-6-fluoro-1-ethyl-1,4-dihydro-4-oxoquinoline-3-carboxylic acid corresponding to the general formula I as claimed in claim 1.

4. 7-(1-Pyrrolyl)-1-ethyl-1,4-dihydro-4-oxo-1,8-naphthyridine-3-carboxylic acid corresponding to the general formula I as claimed in claim 1.

5.7-(1-Pyrrolyl)-6-fluoro-1-ethyl-1,4-dihydro-4-oxo-1,8-naphthyridine-3-carboxylic acid corresponding to the general formula I as claimed in claim 1.

6. Process for the preparation of the derivatives of general formula I such as defined in one of claims 1 to 5, wherein a compound of general formula II

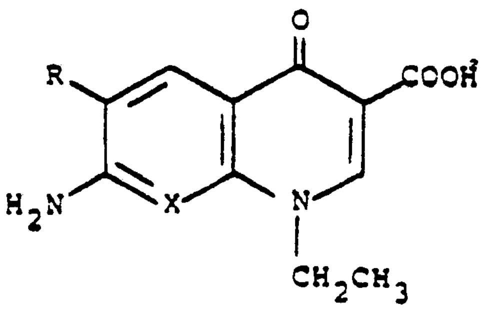

(II)

in which the symbols X and R have the meanings given in claim 1, is caused to react with 2,5-dimethoxytetrahydrofuran in acetic acid medium.

7. By way of novel drugs, particularly as antibacterial and fungicidal drugs, the derivatives of general formula I as claimed in one of claims 1 to 5.

8. Pharmaceutical compositions, which contain, in addition to a pharmaceutically acceptable vehicle, at least one derivative of general formula I as claimed in one of claims 1 to 5.

**Claims** for the vontracting State: AT

1. Process for the preparation of derivatives of 7-(1-pyrrolyl)-1-ethyl-1,4-dihydro-4-oxoquinoline-3-carboxylic acid and 7-(-1-pyrroyl)-1-ethyl-1,4-dihydro-4-oxo-1,8-naphthyridine-3-carboxylic acid according to the general formula I :

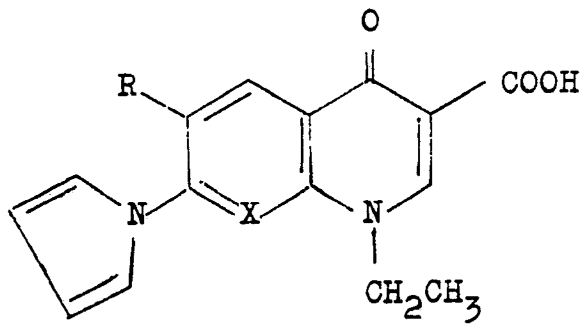

(I)

in which

X represents the CH group or a nitrogen atom, and

R represents a hydrogen atom or a fluorine atom, as well as their physiologically acceptable alkali metal salts or alkaline earth metal salts, wherein a compound of general formula II :

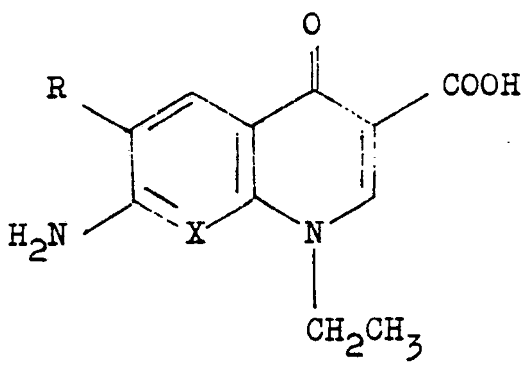

(II)

in which the symbols X and R have the meanings given in claim 1, is caused to react with 2,5-dimethoxytetrahydrofuran in acetic acid medium.

2. Process according to claim 1 in which 7-(-1-pyrrolyl)-1-ethyl-1,4-dihydro-4-oxoquinoline-3-carboxylic acid corresponding to the general formula I as claimed in claim 1 is prepared.

3. Process according to claim 1 in which 7-(-1-pyrrolyl)-6-fluoro-1-ethyl-1,4-dihydro-4-oxoquinoline-3-carboxylic acid corresponding to the general formula I as claimed in claim 1 is prepared.

4. Process according to claim 1 in which 7-(1-pyrrolyl)-1-ethyl-1,4-dihydro-4-oxo-1,8-naphthyridine-3-carboxylic acid corresponding to the general formula I as claimed in claim 1 is prepared.

5. Process according to claim 1 in which 7-(1-pyrrolyl)-6-fluoro-1-ethyl-1,4-dihydro-4-oxo-1,8-naphthyridine-3-carboxylic acid corresponding to the general formula I as claimed in claim 1 is prepared.